(19) Europäisches Patentamt / European Patent Office / Office européen des brevets

(11) **EP 2 982 395 B1**

(12) **EUROPEAN PATENT SPECIFICATION**

(45) Date of publication and mention
of the grant of the patent:
**19.09.2018 Bulletin 2018/38**

(51) Int Cl.:
**A61M 1/02** *(2006.01)*     **A61J 1/05** *(2006.01)*
**A61M 1/34** *(2006.01)*

(21) Application number: **13881392.8**

(22) Date of filing: **01.04.2013**

(86) International application number:
**PCT/JP2013/059876**

(87) International publication number:
**WO 2014/162413 (09.10.2014 Gazette 2014/41)**

(54) **BLOOD BAG SYSTEM**

BLUTBEUTELSYSTEM

SYSTÈME DE POCHES À SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**10.02.2016 Bulletin 2016/06**

(73) Proprietor: **Terumo Kabushiki Kaisha
Tokyo 151-0072 (JP)**

(72) Inventors:
• **HOSOE, Kaoru
Fujinomiya-shi
Shizuoka 418-0004 (JP)**

• **AKIYAMA, Masahiro
Fujinomiya-shi
Shizuoka 418-0004 (JP)**

(74) Representative: **Casalonga
Casalonga & Partners
Bayerstraße 71/73
80335 München (DE)**

(56) References cited:
**GB-A- 2 473 621        JP-A- H11 216 179
JP-A- 2003 530 171    JP-A- 2008 506 482
JP-A- 2009 517 126    JP-B2- H0 540 585
US-A- 5 302 299        US-A- 5 472 621
US-B2- 7 678 272**

EP 2 982 395 B1

**Description**

Technical Field

The present invention relates to a blood bag system for producing a blood component preparation used for transfusion.

Background Art

Conventional blood component preparations include red blood cells, platelet concentrated plasma, platelet poor plasma, and platelet rich plasma. A blood component preparation is produced by performing centrifugal separation of the blood from which a certain blood cell component, such as white blood cells, is removed after sampled from a donor. Such a blood bag system used for producing a blood component preparation is disclosed in, for example, Patent Literature 1 as a sterilization system.

The sterilization system disclosed in Patent Literature 1 includes a first flexible container for receiving blood from a donor, a filter in which a housing is separated into two chambers by a filter, a second flexible container for receiving blood reduced through the filter, a first fluid line section guided from the first flexible container to the filter, and a second fluid line section guided from the filter to the second flexible container. The sterilization system is configured that the first flexible container includes a predetermined amount of air so that no blood is left in the filter and the fluid line. Furthermore, the sterilization system includes a third flexible container provided in the second fluid line section to deaerate the second flexible container.

Citation List

Patent Literature

Document GB 2 473 621.
Document US 7 678 272.
Patent Literature 1: JP 2008-506482 A

Summary of Invention

Technical Problem

However, in the technique disclosed in Patent Literature 1, the predetermined amount of air included in the first flexible container is determined to be larger than the volume in the tube line adjacent the filter. So that the first flexible container includes an excess volume of air which is the sum of the volume in the filter and the volume of the first and the second fluid line sections. Moreover, since the volume in the third flexible container provided in the second fluid line section is also included in the predetermined amount of air, the amount of air included in the first flexible container is further excessive.

An excess amount of air mixes into the blood which is being transferred from the first flexible container to the filter. When filtering the blood including air mixed therein with the filter material, an air block may occur in the filter material to inhibit blood filtration, thereby causing blood to remain in the filter. This reduces the amount of filtered blood to be collected in the second flexible container, which disadvantageously reduces the yield of the blood component preparation which is produced using the filtered blood.

Moreover, mixture of an excess amount of air into the blood to be filtered and stored in the first flexible container induces blood activity, which results in the deterioration in quality of the unfiltered blood during storage. Furthermore, mixture of an excess amount of air into the filtered blood collected in the second flexible container induces blood activity, which results in the deterioration in quality of the filtered blood during storage. As a result, the control of production of the blood component preparation using filtered blood becomes difficult, which disadvantageously results in the decrease in the yield of blood component preparation.

Such a problem may be solved by providing a vent and a gas ejecting port on the inlet and the outlet of the filter or providing a bypass line connecting the first and the second fluid line sections to bypass the filter. However, this may disadvantageously deteriorate operability of blood filtering.

The present invention is directed to solve the aforementioned problem, and the object thereof is to provide a blood bag system that avoids blood remaining in the filter when filtering blood, has high operability, and achieves high yield of the blood component preparation produced from filtered blood.

Solution to Problem

To solve the aforementioned problem, a blood bag system according to the present invention includes a first bag for storing blood sampled from a donor, a first tube for transferring blood from the first bag, a filter including a housing and a filter material separating the inside of the housing into a blood inflow chamber and a blood outflow chamber, blood transferred from the first tube to the blood inflow chamber being reduced in the filter material to reduce a predetermined blood cell component, filtered blood flowing out from the blood outflow chamber;
a second tube for transferring filtered blood flowing out from the blood outflow chamber; and

a second bag for storing filtered blood transferred from the second tube, wherein
the first bag contains a predetermined amount of air satisfying Equation (1) expressed as follows: $(V1 + V2 + V3) \leq$ (amount of air) $< (V1 + V2 + V3 + V4 + V5)$ Equation (1),
where $V1$ is a volume in the first tube, $V2$ is a volume of the blood inflow chamber, $V3$ is a void volume of the filter material, $V4$ is a volume of the blood outflow chamber, and $V5$ is a volume in the second tube.

The blood bag system may further include a third bag and a fourth bag which are coupled to the second bag via coupling tubes.
In the blood bag system according to the present invention, the amount of air in the first bag is appropriate when Equation (1) is satisfied and blood filtering can be performed with no air block occurring in the filter material. Furthermore, the air in the first bag is enclosed in the filter after filtration so that the blood that has remained in the filter can be pushed out to the second tube to be collected in the second bag. This avoids blood remaining in the filter when filtering blood, and the amount of blood collected in the second bag increases. Consequently, the yield of the blood component preparation produced from the collected filtered blood improves.
Furthermore, no air mixes into blood before and after filtration, because the amount of air is not excessive. As a result, favorable quality of blood is kept during storage, because blood activity is not induced before and after filtration. Therefore, the yield of a blood component preparation produced from the collected filtered blood improves. Furthermore, the operability of blood filtering improves, because the vent and the gas ejecting port, or the bypass line is not required.

Advantageous Effects of Invention

The blood bag system according to the present invention avoids blood remaining in the filter when filtering blood, has high operability, and achieves high yield of the blood component preparation produced from filtered blood.

Brief Description of Drawings

Fig. 1 is a schematic view illustrating a configuration of a blood bag system according to the present invention.
Fig. 2 is a cross sectional view of a filter in Fig. 1 taken along the flow direction of blood.

Description of Embodiments

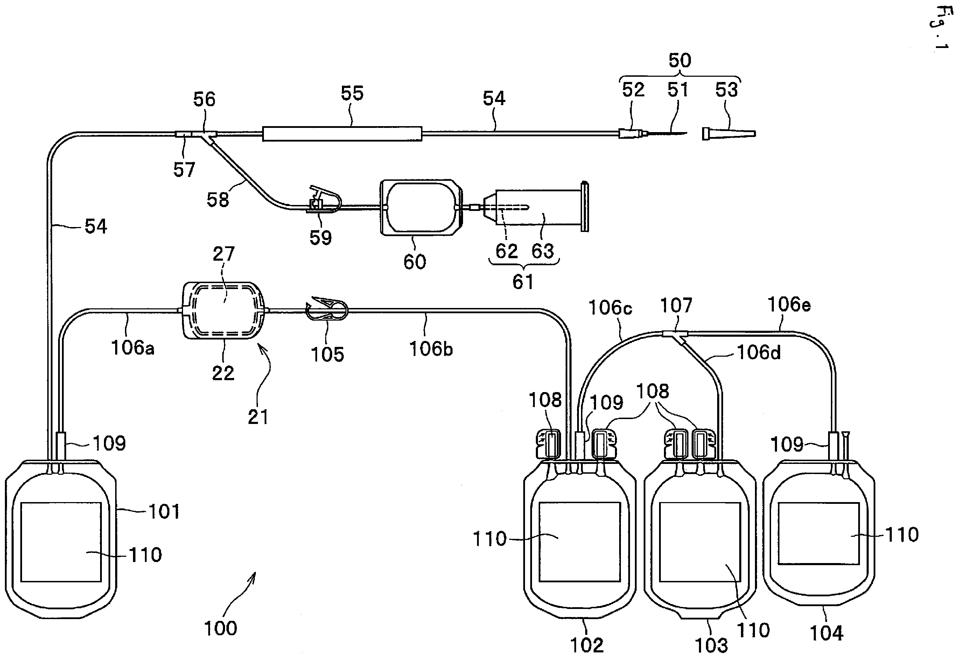

An embodiment of a blood bag system according to the present invention will now be described in detail.
As illustrated in Fig. 1, a blood bag system 100 includes a first bag 101, a first tube 106a, a filter 21, a second tube 106b, and a second bag 102.

(First Bag)

The first bag 101 is a bag container to store the blood sampled from a donor and is produced in a form of a bag by welding the periphery of laminated sheets made of a flexible resin, such as polyvinyl chloride. The first bag 101 can contain a volume of 200 to 1000 ml. Preferably, the first bag 101 is previously filled with an anticoagulant, such as ACD liquid and CPD liquid.
The first bag 101 contains a predetermined amount of air satisfying Equation (1) expressed below. Therefore, no air block occurs in the filter material 27 of the filter 21 when filtering blood, and no blood remains in the filter 21 after the filtration.

$$(V1 + V2 + V3) \leq (\text{amount of air}) < (V1 + V2 + V3 + V4 + V5) \quad \text{Equation (1)}$$

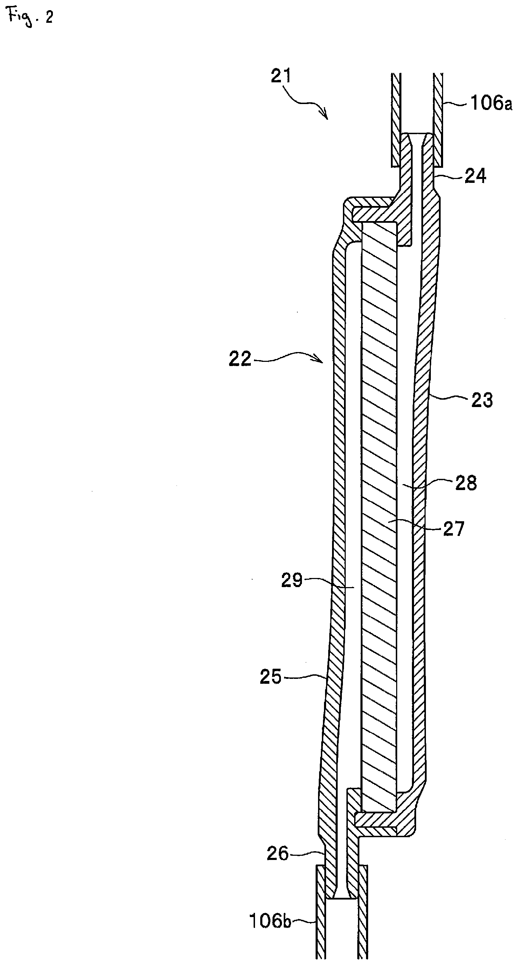

In Equation (1), V1 is a volume in the first tube 106a, V2 is a volume of the blood inflow chamber 28 of the filter 21, V3 is a void volume of the filter material 27 of the filter 21, V4 is a volume of the blood outflow chamber 29 of the filter 21, and V5 is a volume in the second tube 106b (see Fig. 2). The volume V2 of the blood inflow chamber 28 includes the volume in the inflow port 24. The volume V4 of the blood outflow chamber 29 includes the volume in the outflow port 26. The filter 21, the first tube 106a, and the second tube 106b will be described in detail later.

The effect cannot be obtained when the amount of air is below the lower limit of Equation (1). When the amount of air is above the upper limit of the Equation (1), air mixes into the blood which is being transferred from the first bag 101 to the filter 21. The mixed air causes an air block in the filter material 27 when filtering blood, thereby discouraging blood filtering and allowing blood to remain in the filter. Meanwhile, the air mixes into the unfiltered blood stored in the first bag 101 and the filtered blood collected in the second bag 102 to induce blood activity, which deteriorates the quality of blood, before and after being filtered, during storage.

The amount of air in the first bag 101 is adjusted during the manufacturing of the blood bag system 100. Although not shown in the drawing, the blood bag system 100 may further include an air bag filled with air and coupled to the first bag 101 to enclose a predetermined amount of air from the air bag in the first bag 101 after collecting blood into the first bag 101 via the blood sampling tube 54.

A conventionally known blood sampling means is coupled to the first bag 101. The blood sampling means is configured that a blood sampling tube 54 is coupled to the inside of the first bag 101 to supply blood sampled from a donor, and a piercing tool 50 to be pierced into a donor to sample blood is coupled to the distal end of the blood sampling tube 54. The piercing tool 50 includes a blood sampling needle 51, a hub 52 that secures the blood sampling needle 51, and a protector 53 for covering a needle tip of the blood sampling needle 51. The blood sampling means may include a test blood bag 60 used for sampling and storing initial flow blood during blood sampling and a conventionally known accidental piercing prevention tool 55 for preventing accidentally piercing the blood sampling needle 51 after blood sampling.

A conventionally known test blood bag is used as the test blood bag 60 which is coupled to a branching connector 56 provided midway the blood sampling tube 54 via a branching tube 58. A clamp 59 is provided on the branching tube 58 to choke the flow passage of the branching tube 58. In the side to the first bag 101 of the branching connector 56, a breakable seal member 57 is provided to prevent initial flow blood from flowing toward the first bag when sampling initial flow blood. The breakable seal member 57 includes a cover tube and a tube body accommodating a solid distal portion so as to block the flow passage. The breakable seal member 57 is configured to open the flow passage by breaking the solid distal portion in the tube body. A sampling port 61 is coupled to the test blood bag 60, and test blood is collected in a depressurized blood sampling tube (not shown) via the sampling port 61. The sampling port 61 includes a needle assembly 62 and a holder 63 coupled to the needle assembly 62 by accommodating the depressurized blood sampling tube.

(First Tube)

The first tube 106a is for transferring blood from the first bag 101 to the filter 21. One end of the first tube 106a is coupled to the first bag 101 and the other end is coupled to the inflow port 24 (see Fig. 2) of the filter 21. One end of the first tube 106a is coupled to the first bag 101 via a breakable seal member 109 having the same configuration as the breakable seal member 57. The first tube 106a has a volume V1 which is the volume in the tube from the first bag 101 to the filter 21. A conventionally known tube for transferring blood is used as the first tube 106a. For example, a polyvinyl chloride tube made of the same material as the first bag 101 is preferably used.

(Filter)

The filter 21 filters the blood transferred from the first bag 101 via the first tube 106a to remove a predetermined blood cell component, for example, white blood cells or white blood cells and platelets. As illustrated in Fig. 2, the filter 21 includes a housing 22 and the filter material 27 separating the inside of the housing 22 into the blood inflow chamber 28 and the blood outflow chamber 29. In the filter 21, the blood transferred from the first tube 106a to the blood inflow chamber 28 is filtered through the filter material 27, and the filtered blood from which a predetermined blood cell component has been removed flows out from the blood outflow chamber 29.

The housing 22 has an approximately circular (including elliptical) cross section or an approximately rectangular cross section along the flow direction of blood. The housing 22 includes a bottom section 23 made of polycarbonate, polyvinyl chloride, or the like and a cover section 25 made of the same material and having the same shape as the bottom section 23. The bottom section 23 includes the blood inflow chamber 28 having a volume V2 and the inflow port 24 provided on the rim of the bottom section 23 to communicate with the blood inflow chamber 28. The volume V2 of the blood inflow chamber 28 includes the volume in the inflow port 24. The cover section 25 includes the blood outflow chamber 29 having a volume V4 and the outflow port 26 provided on the rim of the cover section 25 to communicate with the blood outflow chamber 29. The volume V4 of the blood outflow chamber 29 includes the volume in the outflow port 26. Considering that the housing 22 deforms by, for example, expansion by introducing blood therein, the volume V2 and the volume V4 of the blood inflow chamber 28 and the blood outflow chamber 29 are of values when blood is not yet introduced in the filter 21.

The filter material 27 includes a porous membrane made of, for example, polyurethane, or an unwoven fabric made of, for example, polyethylene terephthalate having a void volume V3. The void ratio of a porous membrane or an unwoven fabric is preferably 40 to 99%. The filter material 27 preferably has a predetermined zeta potential on the surface to improve removal ratio of a predetermined blood cell component. The filter 21 is manufactured by positioning the filter material 27 between the bottom section 23 and the cover section 25, and welding the rim of the bottom section 23 and the cover section 25 by, for example, ultrasonic or high frequency welding.

(Second Tube)

As illustrated in Fig. 1, the second tube 106b is for transferring the filtered blood flowing out from the blood outflow chamber 29 of the filter 21 to the second bag 102. One end of the second tube 106b is coupled to the outflow port 26 of the filter 21 and the other end is coupled to the second bag 102. A clamp 105 is provided on the second tube 106b to choke the flow passage of the second tube 106b. The second tube 106b has a volume V5 which is a volume in the tube from the filter 21 to the second bag 102. A conventionally known tube used for transferring blood is used as the second tube 106b. For example, a polyvinyl chloride tube made of the same material as the second bag 102 is preferably used.

(Second Bag)

The second bag 102 is a bag container to store the filtered blood transferred from the second tube 106b and has a form of a bag similar to the first bag 101, produced by welding the periphery of laminated sheets made of a flexible resin, such as polyvinyl chloride. The second bag 102 may include a discharge port 108 to discharge outside the filtered blood stored in the second bag 102.

In addition to the configuration described above, the blood bag system 100 according to the present invention may include a third bag 103, a fourth bag 104, and coupling tubes 106c, 106d, and 106e.

(Third Bag)

The third bag 103 is a bag container to store a portion of a blood component separated by centrifugal separation of the filtered blood stored in the second bag 102, and has a form of a bag similar to the first bag 101, produced by welding the periphery of laminated sheets made of a flexible resin, such as polyvinyl chloride. The third bag 103 may include a discharge port 108 to discharge outside the blood component stored in the third bag 103.

(Fourth Bag)

The fourth bag 104 is a bag container to be filled with liquid medicine, such as a red blood cell preservative solution, and has a form of a bag similar to the first bag 101, produced by welding the periphery of laminated sheets made of a flexible resin, such as polyvinyl chloride.

(Coupling Tube)

Coupling tubes 106c, 106d, and 106e are for coupling the third bag 103 and the fourth bag 104 to the second bag 102. The coupling tubes 106d and 106e are coupled, via a branching tube 107, to the coupling tube 106c coupled to the second bag 102. One end of the coupling tube 106c is coupled to the second bag 102, and the other end is coupled to the branching tube 107. The coupling tube 106c is preferably coupled to the second bag 102 via the breakable seal member 109. One end of the coupling tube 106d is coupled to the branching tube 107, and the other end is coupled to the third bag 103. One end of the coupling tube 106e is coupled to the branching tube 107, and the other end is coupled to the fourth bag 104. The coupling tube 106e is preferably coupled to the fourth bag 104 via the breakable seal member 109. A conventionally known tube or pipe is used as the coupling tubes 106c, 106d, and 106e and the branching tube 107. For example, a tube or a pipe made of the same material, that is polyvinyl chloride, as the second bag 102, the third bag 103, and the fourth bag 104 is preferable. The breakable seal member 109 is as described above.

The blood bag system 100 is preferably provided with a label 110 attached to the front side of each of the first bag 101, the second bag 102, the third bag 103, and the fourth bag 104 to indicate the content of the bag.

A method for using the blood bag system will now be described referring to a method of processing blood using the blood bag system 100 illustrated in Fig. 1 as an example.

(1) The accidental piercing prevention tool 55 is positioned to be separated from the hub 52 with the clamp 59 kept opened to keep the branching tube 58 open. Under this state, the blood sampling needle 51 pierces a blood vessel of a donor to start blood sampling. Initial flow blood is collected in the test blood bag 60 via the blood sampling tube 54 and the branching tube 58.

(2) After a predetermined amount of initial flow blood is collected in the test blood bag 60, the clamp 59 is closed to choke the branching tube 58, and the breakable seal member 57 is broken, thereby opening the flow passage, to open the blood sampling tube 54. Then the blood without initial flow blood is collected in the first bag 101 via the blood sampling tube 54.

(3) When a predetermined amount of blood is collected in the first bag 101, the blood sampling needle 51 is pulled out from the blood vessel of the donor and the blood sampling finishes.

(4) The accidental piercing prevention tool 55 is moved toward the hub 52 or the blood sampling tube 54 is pulled toward the first bag 101 to retreat the hub 52 (blood sampling needle 51), so that the piercing tool 50 is accommodated and held in the accidental piercing prevention tool 55.

(5) The blood sampling tube 54 and the branching tube 58 are sealed by welding using a tube sealer or the like. Then the piercing tool 50 and the test blood bag 60 are decoupled from the first bag 101, and the piercing tool 50 is disposed.

(6) The breakable seal member 109 of the first tube 106a is broken to open the flow passage, and the blood collected in the first bag 101 is transferred to the filter 21 via the first tube 106a. Then the filter 21 reduces white blood cells and platelets from the transferred blood. The clamp 105 of the second tube 106b is opened to transfer the filtered blood to the second bag 102 via the second tube 106b to be collected.

(7) The air in the first bag 101 is enclosed in the filter 21 via the first tube 106a by pressing the first bag 101, for example, by hand. Alternatively, the filter 21 may be positioned higher than the first bag 101 to give a difference in elevation (head of fluid) between the filter 21 and the first bag 101, so that by an atmos (pheric pressure presses) the first bag 101, it can transfer the air in the first bag 101 to the filter 21 via the first tube 106a. A conventionally known pressing device may be used. In this manner, the blood remained in the filter 21 is collected in the second bag 102 via the second tube 106b.

(8) The clamp 105 of the second tube 106b is set to the choking state, and the second tube 106b is sealed by welding using a tube sealer or the like. The first bag 101 and the filter 21 are decoupled from the second bag 102.

(9) Centrifugal separation of the second bag 102 is performed to separate the blood in the bag into a red blood cell layer and a plasma layer. The breakable seal member 109 of the coupling tube 106c is broken to open the flow passage, so that plasma is transferred to the third bag 103 via the coupling tubes 106c and 106d. Then the red blood cell preservative solution is supplied from the fourth bag 104 via the coupling tubes 106c and 106e to be added and mixed in the concentrated blood cells remaining in the second bag 102. As a result, red blood cell is prepared in the second bag 102 and platelet poor plasma is prepared in the third bag 103.

(10) In step (6), when only white blood cells are reduced in the filter 21, platelet rich plasma is prepared in the third bag 103 in step (9). In this case, centrifugal separation may be performed for the third bag 103, so that platelet concentrated plasma is prepared in the third bag 103 and platelet poor plasma is prepared in the fourth bag 104 which has been empty after transferring red blood cell preservative solution therefrom.

Reference Signs List

| 21 | filter |
| 22 | housing |
| 27 | filter material |
| 28 | blood inflow chamber |
| 29 | blood outflow chamber |
| 100 | blood bag system |
| 101 | first bag |
| 102 | second bag |

| 103 | third bag |
| 04 | fourth bag |
| 106a | first tube |
| 106b | second tube |
| 106c | coupling tube |
| 106d | coupling tube |
| 106e | coupling tube |

**Claims**

1. A blood bag system (100) comprising:

   a first bag (101) for storing blood sampled from a donor;
   a first tube (106a) for transferring blood from the first bag (101);
   a filter (21) including a housing (22) and a filter material (27) separating inside of the housing (22) into a blood inflow chamber (28) and a blood outflow chamber (29), blood transferred from the first tube (106a) to the blood inflow chamber (28) being reduced in the filter material (27) to remove a predetermined blood cell component, filtered blood flowing out from the blood outflow chamber (29);
   a second tube (106b) for transferring filtered blood flowing out from the blood outflow chamber (29); and
   a second bag (102) for storing filtered blood transferred from the second tube (106b), **characterised by** that the first bag (101) contains a predetermined amount of air satisfying Equation (1) expressed as follows: $(V1 + V2 + V3) \leq$ (amount of air) $< (V1 + V2 + V3 + V4 + V5)$ Equation (1),
   where V1 is a volume in the first tube (106a), V2 is a volume of the blood inflow chamber (28), V3 is a void volume of the filter material (27), V4 is a volume of the blood outflow chamber (29), and V5 is a volume in the second tube (106b).

2. The blood bag system (100) according to claim 1 further comprising a third bag (103) and a fourth (104) bag coupled to the second bag (102) via coupling tubes (106c, 106d, 106e).

**Patentansprüche**

1. Blutbeutelsystem (100), umfassend:

   einen ersten Beutel (101) zur Aufbewahrung von Blut, das einem Spender entnommen wurde;
   einen ersten Schlauch (106a) zum Übertragen von Blut aus dem ersten Beutel (101);
   einen Filter (21) mit einem Gehäuse (22) und einem Filtermaterial (27), das die Innenseite des Gehäuses (22) in eine Bluteinströmkammer (28) und eine Blutausströmkammer (29) auftrennt, wobei Blut, das von dem ersten Schlauch (106a) zu der Bluteinströmkammer (28) übertragen wird, in dem Filtermaterial (27) reduziert wird, um eine vorbestimmte Blutzellenkomponente zu entfernen, wobei gefiltertes Blut aus der Blutausströmkammer (29) ausströmt;
   einen zweiten Schlauch (106b) zum Übertragen von gefiltertem Blut, das aus der Blutausströmkammer (29) ausströmt; und
   einen zweiten Beutel (102) zum Aufbewahren von gefiltertem Blut, das aus dem zweiten Schlauch (106b) übertragen wird,
   **dadurch gekennzeichnet, dass** der erste Beutel (101) eine vorbestimmte Menge an Luft enthält, die Gleichung (1) erfüllt, die wie folgt angegeben ist:

   $(V1 + V2 + V3) \leq$ (Luftmenge) $< (V1 + V2 + V3 + V4 + V5)$ Gleichung (1),
   wobei V1 ein Volumen in dem ersten Schlauch (106a) ist, V2 ein Volumen der Bluteinströmkammer (28) ist, V3 ein Hohlraumvolumen des Filtermaterials (27) ist,
   V4 ein Volumen der Blutausströmkammer (29) ist und V5 ein Volumen in dem zweiten Schlauch (106b) ist.

2. Blutbeutelsystem (100) nach Anspruch 1, ferner umfassend einen dritten Beutel (103) und einen vierten Beutel (104), die über Verbindungsschläuche (106c, 106d, 106e) mit dem zweiten Beutel (102) verbunden sind.

**Revendications**

1.  Système de poches à sang (100) comprenant :

     une première poche (101) destinée à stocker du sang prélevé chez un donneur ;
     un premier tube (106a) pour transférer le sang de la première poche (101) ;
     un filtre (21) comprenant un boîtier (22) et un matériau filtrant (27) séparant l'intérieur du boîtier (22) en une chambre d'entrée de sang (28) et une chambre de sortie de sang (29), le sang transféré du premier tube (106a) vers la chambre d'entrée de sang (28) étant réduit dans le matériau filtrant (27) pour retirer un composant de cellule sanguine prédéterminé, le sang filtré sortant de la chambre de sortie de sang (29) ;
     un second tube (106b) pour transférer le sang filtré sortant de la chambre de sortie de sang (29) ; et
     une seconde poche (102) destinée à stocker le sang transféré du second tube (106b),
     **caractérisé en ce que** la première poche (101) contient une quantité prédéterminée d'air répondant à l'Équation (1) exprimée comme suit : $(V1 + V2 + V3) \leq$ (quantité d'air) $< (V1 + V2 + V3 + V4 + V5)$ Équation (1),
     où V1 est un volume dans le premier tube (106a), V2 est un volume de la chambre d'entrée de sang (28), V3 est un volume de vide du matériau filtrant (27), V4 est un volume de la chambre de sortie de sang (29), et V5 est un volume dans le second tube (106b).

2.  Système de poches à sang (100) selon la revendication 1, comprenant en outre une troisième poche (103) et une quatrième poche (104) couplées à la seconde poche (102) par l'intermédiaire de tubes de couplage (106c, 106d, 106e).

Fig.1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2473621 A
- US 7678272 B
- JP 2008506482 A